# EUROPÄISCHE PATENTSCHRIFT

(11) **EP 0 332 042 B1**
(45) Veröffentlichungstag und Bekanntmachung des Hinweises auf die Patenterteilung: **25.05.1994**
(21) Anmeldenummer: 89103607.1
(22) Anmeldetag: 02.03.1989
(51) Int. Cl.: C07D 251/24, G03F 7/027, G03C 1/72, G03F 7/004

(54) **Durch 4,6-Bis-trichlormethyl-s-triazin-2-ylgruppen substituierte aromatische Verbindungen, Verfahren zu ihrer Herstellung und ihre Verwendung in lichtempfindlichen Gemischen**
Aromatic compounds substituted by 4,6-bis-trichloromethyl-s-triazin-2-yl groups, process for their preparation and their use in photosensitive mixtures
Composés aromatiques substitués par des groupes 4,6-bis-trichlorométhyl-s-triazin-2-yles, procédé pour leur préparation et leur utilisation dans des mélanges photosensibles

(30) Priorität: 07.03.1988 DE 3807378
(43) Veröffentlichungstag der Anmeldung: 13.09.1989
(73) Patentinhaber: HOECHST AKTIENGESELLSCHAFT, 65926 Frankfurt am Main (DE)
(72) Erfinder: Pawlowski, Georg, Dr., D-6200 Wiesbaden (DE); Erdman, Fritz, Dr., D-6228, Eltville 4 (DE); Lutz, Heidrun,, D-6500 Mainz (DE)

(56) Entgegenhaltungen:
- EP-A- 0 137 452
- US-A- 4 189 323

## Beschreibung

Die Erfindung betrifft aromatische 4,6-Bis-trichlormethyl-s-triazin-2-yl-verbindungen, Verfahren zu ihrer Herstellung und ihre Verwendung als Photoinitiatoren in lichtempfindlichen Gemischen. Die erfindungsgemäßen Verbindungen sind ferner wertvolle Zwischenprodukte zur Herstellung von hochaktiven Photoinitiatoren oder photolytisch wirksamen Säurespendern für lichtempfindliche Gemische.

4,6-Bis-trichlormethyl-s-triazin-2-yl-phenylverbindungen sind in zahlreichen Ausführungsformen bekannt; eine ausführliche Darstellung verschiedener Verbindungen dieses Typs wird von M. Wakabayashi et al., Bull. Chem. Soc. Jpn., 42, 2924 (1969) gegeben. Der Phenylkern kann dabei durch verhältnismäßig inerte chemische Gruppierungen, wie z. B. Nitro-, Alkyl- oder Alkoxygruppen oder Halogenatome substituiert sein.

Aus der WO 81/02261 sind lichtempfindliche Mischungen bekannt, die als Photoinitiatoren 4,6-Bis-trichlormethyl-s-triazin-2-yl-phenylderivate enthalten, in denen der aromatische Kern durch 1 bis 3 Alkyl- oder Alkoxygruppen substituiert ist. Die dort beschriebenen lichtempfindlichen Mischungen werden zur Herstellung von druckempfindlichen Klebstreifen verwendet. Die Photoaktivität dieser Verbindungen liegt im nahen UV-Bereich.

Die vorstehend genannten 4,6-Bis-trichlormethyl-s-triazin-2-yl-phenylderivate haben als Zwischenprodukte praktisch keine Bedeutung, da sie sich nicht, oder nur unter zumindest partieller Zerstörung der Trichlormethylgruppen weiter umsetzen lassen.

Andererseits ist bekannt, daß bestimmte 4,6-Bis-trichlormethyl-s-triazin-2-yl-derivate hervorragende Eigenschaften als Photoinitiatoren und photolytische Säurespender aufweisen. Beispiele für derartige Verbindungen sind aus der DE-C 27 18 259 bekannt, worin ggf. substituierte 4,6-Bis-trichlormethyl-s-triazin-2-yl-naphthylderivate beschrieben werden, oder der EP-A 0 137 452, aus der ggf. substituierte 4,6-Bis-trichlormethyl-s-triazin-2-yl-styrylphenylderivate als Photoinitiatoren hervorgehen. Obwohl die hier genannten Verbindungen im nahen UV-Bereich und teilweise auch im sichtbaren Bereich hochaktiv sind, haben sie den Nachteil, daß ihre Herstellung häufig sehr kompliziert, kostenintensiv und zeitaufwendig ist.

Es besteht daher ein Bedarf für neue 4,6-Bis-trichlormethyl-s-triazin-2-yl-derivate, die sich mit vergleichsweise geringem synthetischem Aufwand und unter Erhalt der Trichlormethylgruppen in chromophorsubstituierte Verbindungen überführen lassen, die im nahen UV-Bereich oder im sichtbaren Bereich hohe Photoaktivitäten als Initiatoren in photopolymerisierbaren Mischungen oder als Säurespender in säurespaltbaren Gemischen aufweisen.

Aufgabe der Erfindung war es daher, neue 4,6-Bis-trichlormethyl-s-triazin-2yl-phenylderivate bereitzustellen, die einfach und kostengünstig herstellbar sind, und die sich mit geringem Aufwand in chromophor-substituierte Photoinitiatoren umwandeln lassen, die in technisch interessierenden Bereichen des elektromagnetischen Spektrums hohe Aktivitäten aufweisen.

Gegenstand der Erfindung sind Verbindungen der allgemeinen Formel I 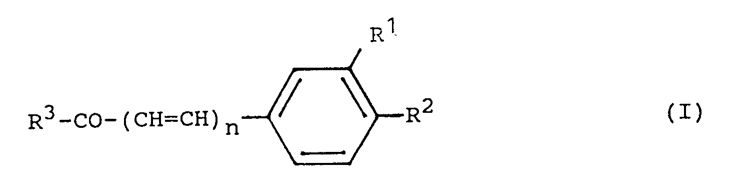
worin
- R¹ und R²: voneinander verschieden sind und entweder ein Wasserstoffatom oder einen Rest der Formel 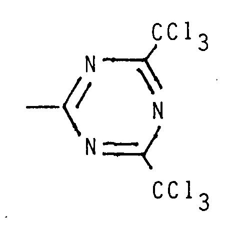
- R³: einen ggf. durch Halogenatome oder Arylgruppen substituierten Alkylrest mit 1 bis 16 Kohlenstoffatomen, einen Alkenyloxy- oder Alkinyloxyrest mit 2 bis 6 Kohlenstoffatomen, einen Alkoxyalkoxyrest mit 3 bis 10 Kohlenstoffatomen, einen ggf. durch Halogenatome, Alkyl-, Alkoxy- oder Nitrogruppen substituierten Aryloxyrest mit 6 bis 14 Kohlenstoffatomen oder einen Aryloxyalkoxyrest mit 8 bis 12 Kohlenstoffatomen, eine Hydroxygruppe oder ein Halogenatom und
- n: die Zahl 0 oder 1
bedeuten.

Erfindungsgemäß wird ferner ein Verfahren zur Herstellung von Verbindungen der vorstehend angegebenen Formel I vorgeschlagen, das darin besteht, daß man eine Verbindung der Formel II 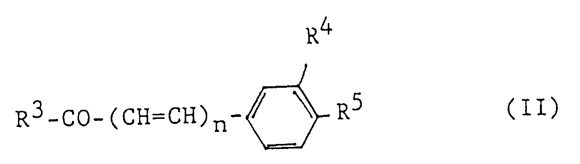
worin R³ und n die oben angegebene Bedeutung haben und R⁴ und R⁵ voneinander verschieden sind und entweder ein Wasserstoffatom oder eine CN-Gruppe bedeuten, mit Trichloracetonitril unter Einwirkung von HCl-Gas in Gegenwart einer Lewis-Säure umsetzt.

Die erfindungsgemäßen Verbindungen sind lichtempfindlich und eignen sich als Photoinitiatoren bzw. als photolytische Säurespender in lichtempfindlichen Gemischen. Hierfür werden die Verbindungen der Formel I bevorzugt, in denen R³ einen ggf. substituierten Alkoxy-, Alkenyloxy-, Alkinyloxy- oder Aryloxyest bedeutet. Von diesen sind besonders die Verbindungen mit n = 1 geeignet.

Von den Verbindungen der Formel I sind insbesondere diejenigen mit R³ = Halogen wertvolle Zwischenprodukte zur Herstellung von lichtempfindlichen Verbindungen mit hoher Aktivität im sichtbaren und nahen Ultraviolettbereich des elektromagnetischen Spektrums. Sie lassen sich über die Säurechloridgruppe leicht mit reaktionsfähigen aromatischen, heterocyclischen oder olefinischen Verbindungen unter Ausbildung eines nach Wunsch verlängerten konjugierten Systems umsetzen, wobei die photoaktiven Trichlormethylgruppen am Triazinring erhalten bleiben.

Ein Beispiel für derartige Synthesen ist die Herstellung von Oxadiazolderivaten durch Umsetzen von I mit Verbindungen 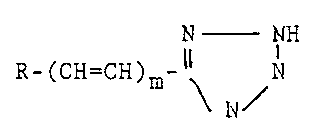
zu Oxadiazolderivaten 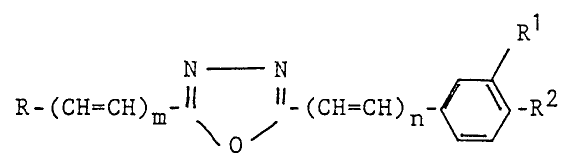
worin R ein ggf. substituierter Aryl- oder Heteroarylrest und m Null oder 1 ist. Diese Verbindungen sind in der gleichzeitig eingereichten EP-A 0332043 näher beschrieben.

Weiterhin können Verbindungen der Formel I mit Verbindungen der Formel 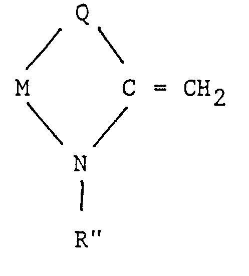
oder deren Iminiumsalz 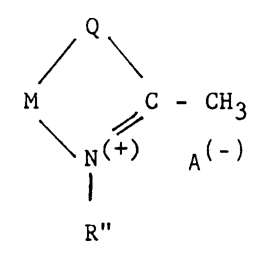
zu Verbindungen der Formel 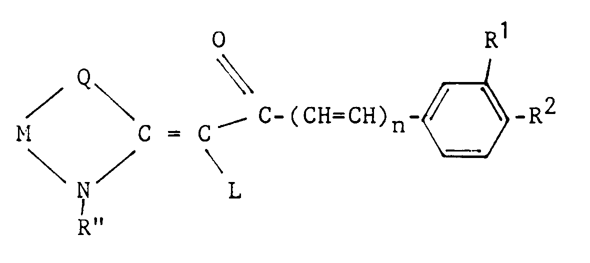
umgesetzt werden, worin
- R'': einen Alkyl-, Aralkyl-, Aryloxyalkyl- oder Alkoxyalkylrest,
- L: ein Wasserstoffatom oder einen Substituenten der Formel 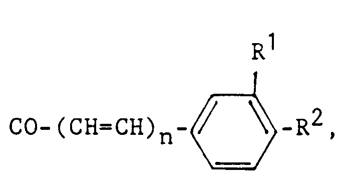
- M: einen substituierten oder unsubstituierten Alkylenrest oder Alkenylenrest oder einen 1,2-Arylenrest und
- Q: ein Schwefel-, Selen- oder Sauerstoffatom, eine Dialkylmethylengruppe, einen Alken-1,2-ylenrest, einen 1,2-Phenylenrest oder eine Gruppe N-R''
bedeuten, wobei M + Q zusammen 3 oder 4 Ringglieder bilden.

Diese Verbindungen sind in der gleichzeitig eingereichten EP-A 0 332 044 näher beschrieben.

Besonders bevorzugt werden von diesen Verbindungen solche, in denen R³ eine Alkoxygruppe mit 1 bis 6 Kohlenstoffatomen, eine Alkenyloxygruppe mit 2 bis 4 Kohlenstoffatomen oder eine Alkinyloxygruppe mit 2 bis 4 Kohlenstoffatomen, eine Hydroxygruppe der ein Chloratom bedeutet.

Wenn R³ ein Halogenatom ist, ist es insbesondere ein Chloratom.

Bevorzugte Verbindungen werden nachstehend namentlich aufgeführt:
3-(4,6-Bis-trichlormethyl-s-triazin-2-yl)-benzoesäure, -benzoesäuremethylester, -benzoesäureethylester, -benzoylchlorid; 4-(4,6-Bis-trichlormethyl-s-triazin-2-yl)-benzoesäure, -benzoesäuremethylester, -benzoesäureethylester, -benzoylchlorid; 3-(4,6-Bis-trichlormethyl-s-triazin-2-yl)-zimtsäure, -zimtsäuremethylester, -zimtsäureethylester, -cinnamoylchlorid; 4-(4,6-Bis-trichlormethyl-s-triazin-2-yl)-zimtsäure, -zimtsäuremethylester, -zimtsäureethylester und -cinnamoylchlorid. Unter diesen sind die Verbindungen mit n = O ganz besonders bevorzugt.

Die erfindungsgemäßen Verbindungen der allgemeinen Formel I sind i.a. kristalline, farblose bis gelblich gefärbte Substanzen, die unter Ausschluß von Licht und ggf. Feuchtigkeit praktisch unbegrenzt haltbar sind.

Für die Herstellung der erfindungsgemäßen Verbindungen der allgemeinen Formel I bieten sich verschiedene Verfahrensmöglichkeiten an, die je nach Art der eingesetzten Verbindung unterschiedliche Ergebnisse bezüglich Ausbeute oder Raum-Zeit-Ausbeute liefern.

In dem nachstehend beschriebenen Schema I werden die unterschiedlichen Herstellungsmethoden schematisch dargestellt, wobei R′ für einen der unter R³ aufgeführten organischen Reste steht.

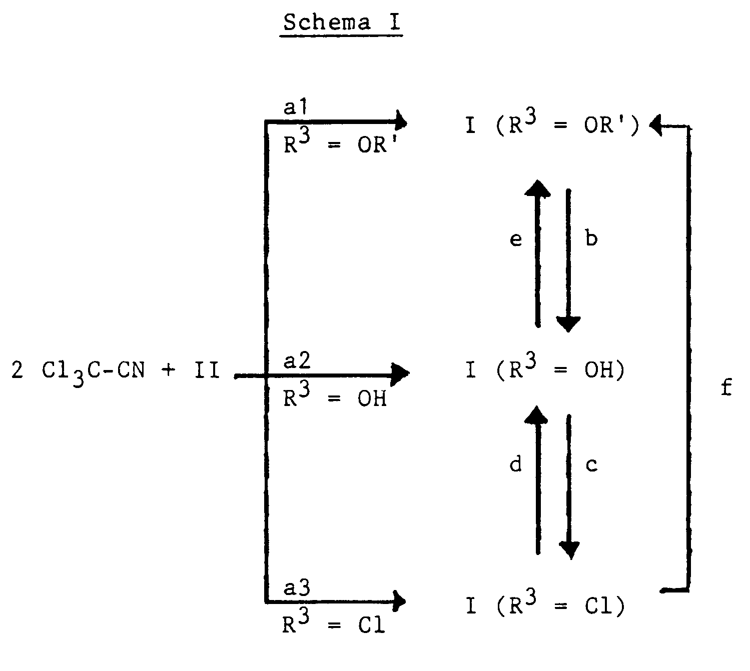

### 1. Herstellungsverfahren für Verbindungen mit R³ = OR′

### Methode a1:

Durch Cotrimerisierung von Trichloracetonitril mit den entsprechenden 3- oder 4-Cyano-benzoe- oder -zimtsäureestern mit Chlorwasserstoffgas in Gegenwart einer Lewis-Säure [analog K. Wakabayashi et al., Bull. Chem. Soc. Jpn., 42, 2924 (1969)].

Der 3- oder 4-Cyano-benzoe- oder -zimtsäureester, bevorzugt der entsprechende Methyl- oder Ethylester wird in einem 2- bis 10-fachen, bevorzugt etwa 6-fachen Überschuß von Trichloracetonitril bei Temperaturen von 10 bis 50 °C, bevorzugt 20 bis 30 °C gelöst oder suspendiert. Ist der Ester nicht in Trichloracetonitril löslich, so kann die Reaktion auch unter Zusatz eines geeigneten, säurestabilen Lösungsmittels, z. B. Dichlormethan, durchgeführt werden. Die Lösung bzw. Suspension wird bei einer Temperatur zwischen -20 und +50 °C, bevorzugt bei 25 °C, unter Feuchtigkeitsausschluß gerührt. Unter ausreichender Kühlung wird eine Lewis-Säure, z. B. Aluminiumchlorid, Bortrichlorid-Etherat, Zinnchlorid oder Zinkchlorid, bevorzugt Aluminiumbromid, in einem Molverhältnis von 1 : 5 bis 1 : 50, bevorzugt um 1 : 10, bezogen auf den Ester, zugefügt und wasserfreier Chlorwasserstoff eingeleitet. Die auftretende Wärmeenergie wird mittels Kühlung abgeführt. Die Chlorwasserstoffeinleitung wird nach 2 bis 20, bevorzugt etwa 3 bis 6 Stunden beendet, und das sich verfestigende Reaktionsprodukt bei Raumtemperatur, gegebenenfalls unter leichter Kühlung, nachreagieren gelassen. Nach 5 bis 100 Stunden ist die Chlorwasserstoffentwicklung abgeklungen und die Reaktion abgeschlossen. Durch Aufnehmen in einem inerten Lösungsmittel, vorzugsweise Dichlormethan, und Ausschütteln mit dem gleichen Volumen Wasser wird der Katalysator und der Säureüberschuß ausgewaschen. Die organische Phase wird getrocknet, eingeengt und das i.a. kristallin anfallende, bereits sehr reine Produkt gegebenenfalls durch geeignete Reinigungsmaßnahmen aufbereitet.

### Methode e:

Die nach Methode a2 hergestellte 3- oder 4-(4,6-Bis-trichlormethyl-s-triazin-2-yl)-benzoe- oder -zimtsäure wird durch extraktive Veresterung in die entsprechenden Ester übergeführt.

Die 3- oder 4-(4,6-Bis-trichlormethyl-s-triazin-2-yl)-benzoe- oder -zimtsäure wird in der 2- bis 50-fachen Menge eines Lösungsmittels, das mit Wasser ein Azeotrop bildet, wie Dichlormethan, Trichlormethan, Toluol oder Xylol, gelöst oder suspendiert. Dazu wird eine Katalysatorsäure, beispielsweise Schwefelsäure, Naphthalinsulfonsäure, ein acides Ionenaustauscherharz oder bevorzugt Toluolsulfonsäure, in einem Molverhältnis von 1 : 100 bis 1 : 10, bevorzugt von 1 : 30 bis 1 : 15, bezogen auf die zu veresternde Säure, gegeben und dem Gemisch der wasserfreie Alkohol, bevorzugt ein Alkohol mit mehr als 1 C-Atom, in einem 1,2- bis 12-fachen, bevorzugt etwa 2-fachen Überschuß zugefügt. Die Mischung wird auf 35 bis 170 °C, bevorzugt 75 bis 140 °C erwärmt, und das gebildete Wasser durch einen geeigneten Abscheider abgetrennt. Die Wasserbildung endet in der Regel nach 2 bis 24 Stunden. Das auf 10 bis 25 °C abgekühlte Gemisch wird mit einer verdünnten schwach alkalisch reagierenden Salzlösung, bevorzugt einer wäßrigen Natriumhydrogencarbonat-Lösung gewaschen und die organische Phase wie vorstehend beschrieben aufgearbeitet.

Zur Herstellung der Methylester wird das Benzoe- oder Zimtsäurederivat in der 2- bis 20-fachen, im allgemeinen etwa 3- bis 8-fachen Menge wasserfreien Methanols suspendiert und mit Schwefelsäure oder bevorzugt Toluolsulfonsäure im oben angegebenen Molverhältnis versetzt. Gegebenenfalls kann ein nicht mit Wasser mischbares, nicht Azeotrope bildendes Lösungsmittel, z. B. ein Dialkylether, oder eines der oben genannten Azeotrope bildenden Lösungsmittel in der 2- bis 10-fachen Menge zugefügt werden. Das Gemisch wird 2 bis 20 Stunden, zumeist etwa 4 bis 8 Stunden auf 30 bis 100 °C erwärmt. Der überschüssige Alkohol wird abgetrennt, der Rückstand in einem nicht mit Wasser mischbaren organischen Lösungsmittel, bevorzugt einem Halogenkohlenwasserstoff oder Ether, aufgenommen und wie vorstehend beschrieben aufgearbeitet.

### Methode f:

Das nach Methode a3 hergestellte 3- oder 4-(4,6-Bis-trichlormethyl-s-triazin-2-yl)-benzoyl- oder -cinnamoylchlorid wird verestert.

Das entsprechende Säurechlorid wird in der 5- bis 20-fachen Menge eines inerten, wasserfreien Lösungsmittels, z. B. Dichlormethan, Toluol oder Tetrahydrofuran, gelöst. Dazu wird in einem 1,1- bis 5-fachen, bevorzugt 1,1 bis 1,5-fachen Überschuß der gewünschte Alkohol oder ein Phenol hinzugefügt. Die Mischung wird bei einer Temperatur zwischen -15 °C und +50 °C gerührt. Handelt es sich bei dem Alkohol um einen ungesättigten Alkohol, so wird ein Temperaturbereich zwischen -15 °C bis + 15 °C bevorzugt; ist der Alkohol gesättigt, so wird ein Temperaturbereich von +5 °C bis 35 °C bevorzugt. Dazu wird ein 1,1- bis 5-facher, bevorzugt 1,1 bis 1,5-facher Überschuß, bezogen auf das Säurechlorid, eines Säurebinders, wie Pyridin oder Triethylamin, zugetropft, wobei die Temperatur mittels Kühlung konstant gehalten wird. Das Gemisch wird 1 bis 10 Stunden, zumeist etwa 3 Stunden bei der vorgegebenen Temperatur nachgerührt und dann auf Raumtemperatur erwärmen bzw. abkühlen gelassen. Die gebildeten Salze werden abgetrennt und die gegebenenfalls mit einem nicht wasserlöslichen Lösungsmittel, z. B. Diethylether, Dichlormethan oder Toluol, verdünnte Mischung wird wie unter Methode e beschrieben aufgearbeitet.

### Herstellungsverfahren für Verbindungen mit R³ = OH

### Methode a2:

Es wird unter Verwendung von 3- oder 4-Cyano-benzoe- bzw. -zimtsäure wie unter Methode a1 beschrieben verfahren. Da die Säuren in Trichloracetonitril häufig unzureichend löslich sind, muß man dann in Suspension oder unter Zusatz eines organischen, inerten Lösungsmittels arbeiten. In beiden Fällen verläuft die Reaktion dann sehr langsam. Das Verfahren hat daher nur begrenzte Bedeutung zur Herstellung der 3- oder 4-(4,6-Bis-trichlormethyl-s-triazin-2-yl)-benzoe- oder -zimtsäurederivate.

### Methode b:

Durch Umesterung von 3- oder 4-(4,6-Bis-trichlormethyl-s-triazin-2-yl)-benzoe- oder -zimtsäuremethylester mit Trichloressigsäure in Gegenwart einer starken Mineralsäure.

Der Methylester, bevorzugt der entsprechende Benzoesäuremethylester, wird mit der 0,5 bis 10-fachen, bevorzugt der etwa 2-fachen Menge an Trichloressigsäure versetzt. Dazu wird eine katalytische Menge, bevorzugt im Verhältnis um 1 : 50, bezogen auf den Ester, einer starken anorganischen Säure, bevorzugt Schwefelsäure, gegeben. Das Gemisch wird auf 130 bis 190 °C, bevorzugt 150 bis 180 °C erwärmt, und der entstehende Trichloressigsäuremethylester wird destillativ abgetrennt. Nachdem 60 bis 90 % der theoretischen Menge übergegangen ist, wird die Mischung auf 50 bis 120 °C, bevorzugt 50 bis 90 °C abkühlen gelassen und in Eiswasser gegeben, wobei die gewünschten Säuren ausfallen. Die erhaltene Mischung wird 10 bis 30 Minuten gerührt, abgenutscht und mit ausreichend Wasser gewaschen. Die erhaltene Säure kann durch Umkristallisieren gereinigt werden.

Durch Überführen von 3- oder 4-(4,6-Bis-trichlormethyl-s-triazin-2-yl)-benzoe- oder -zimtsäuremethyl- oder -ethylester in den entsprechenden Trimethylsilylester und anschließende Hydrolyse.

Der Ester, vorzugsweise ein Methylester, wird in der 2- bis 20-fachen Menge eines inerten, trockenen Lösungsmittels, bevorzugt Chloroform oder 1,2-Dichlorethan, gelöst. Unter Stickstoffatmosphäre wird ein 1- bis 3-facher, bevorzugt etwa 1,5-facher Überschuß an Hexamethyldisilan und anschließend die äquivalente Menge Jod hinzugefügt. Das sich erwärmende Gemisch wird 2 bis 48 Stunden, insbesondere 4 bis 10 Stunden auf eine Temperatur zwischen 60 und 150 °C, bevorzugt um 85 °C gebracht. Die erhaltene bräunliche Mischung wird auf 0 bis 50 °C, bevorzugt 10 bis 20 °C, abkühlen gelassen und mit der 0,5- bis 5-fachen, bevorzugt etwa der gleichen Volumenmenge Eiswasser versetzt. Nach 10- bis 60-minütigem Rühren wird das Lösungsmittel abgedampft und der ausgefallene Rückstand in einem Alkohol, bevorzugt Methanol, digeriert. Das entstandene Produkt wird durch Filtrieren isoliert und gegebenenfalls durch Umkristallisieren gereinigt.

### Methode d:

Durch Hydrolyse des entsprechenden 3- oder 4-(4,6-Bis-trichlormethyl-s-triazin-2-yl)-benzoyl- oder -cinnamoylchlorids. Diese Methode verläuft sehr gut, ist aber ohne praktische Bedeutung, da die entsprechenden Säurechloride bevorzugt aus den Säuren hergestellt werden.

### Herstellungsverfahren für Verbindungen mit R³ = Cl

### Methode a3:

Es wird unter Verwendung von 3- oder 4-Cyano-benzoyl- oder -cinnamoylchlorid wie unter Methode a1 beschrieben verfahren. Wegen der hohen Reaktivität der Säurechloridgruppen ist eine Reinigung der Produkte bei dieser Reaktionsführung nur durch Hochvakuumdestillation möglich. Das Verfahren hat daher geringere praktische Bedeutung.

### Methode c:

Durch Umsetzung der 3- oder 4-(4,6-Bis-trichlormethyl-s-triazin-2-yl)-benzoe- oder -zimtsäure mit Thionylchlorid.

Die entsprechende Säure wird mit der 2- bis 6-fachen, bevorzugt 2,5 bis 4-fachen Gewichtsmenge Thionylchlorid versetzt und auf 50 bis 100, bevorzugt etwa 80 °C, erhitzt, bis keine SO₂-Entwicklung mehr zu beobachten ist. Nach 2 bis 20 Stunden, z. B. etwa 6 Stunden, wird das überschüssige Thionylchlorid abdestilliert. Das im Rückstand enthaltene Säurechlorid kann durch Umkristallisieren gereinigt werden.

Aus diesen Ausführungen läßt sich ersehen, daß der bevorzugte Zugang zu den Esterderivaten der allgemeinen Formel I über die Wege a1, e und f; zu den Säurederivaten über den Weg b und zu den Säurechloriden über den Weg c erfolgt. Die einzelnen Verbindungsklassen lassen sich mit geringem präparativem Aufwand ineinander überführen.

Trotz literaturbekannter analoger Verfahren zur Herstellung von Bis-trichlormethyl-triazinderivaten war das Gelingen der vorstehend beschriebenen Trimerisierungen zu den erfindungsgemäßen Verbindungen der allgemeinen Formel I keineswegs selbstverständlich, da zu befürchten war, daß z. B. die Ester unter den Trimerisierungsbedingungen gespalten werden könnten. Wegen der geringen Löslichkeit der Säuren war bei ihnen keine Cotrimerisierungsneigung zu erwarten. Bei der Verwendung der Säurechloride stand zu befürchten, daß Acylierungsnebenreaktionen ablaufen würden.

Die erfindungsgemäßen Verbindungen der allgemeinen Formel I sind neu und eignen sich in vorteilhafter Weise als Photoinitiatoren in photopolymerisierbaren Mischungen, wobei sie durch geeignete Farbstoffe spektral sensibilisiert werden können. So sind sie z. B. als Coinitiatoren in photopolymerisierbaren Gemischen in Kombination mit bestimmten heterocyclischen Initiatoren und ggf. photoreduzierbaren Farbstoffen geeignet. So können sie z. B. mit Vorteil in photopolymerisierbaren Gemischen aus polymerisierbaren (Meth)acrylsäureestern, photoreduzierbaren Farbstoffen und ggf. Acridin-, Phenazin- oder Chinoxalinverbindungen, wie sie in den älteren EP-A 0 287 817, 0 284 938, 0 321 826, 0 321 827 und 0 321 828 beschrieben sind, anstelle der dort genannten Trichlormethylverbindungen eingesetzt werden.

Die wichtigste Anwendung der erfindungsgemäßen Verbindungen, insbesondere derjenigen mit R³ = Halogen, beruht auf der Tatsache, daß sie als wertvolle Zwischenprodukte für die Herstellung hochaktiver neuer Photoinitiatoren oder photolytischer Säurespender verwendet werden können, beispielsweise von Verbindungen auf Oxadiazolbasis oder auf Basis von Carbonylmethylenheterocyclen, die nach den Verfahren der gleichzeitig eingereichten EP-A 0 332 043 und 0 332 044 herstellbar sind.

Wegen der günstigen Initiatoreigenschaften der erfindungsgemäßen trichlormethylsubstituierten Verbindungen der allgemeinen Formel I, sowie insbesondere des durch sie ermöglichten einfachen Zugangs zu weiteren außerordentlich wertvollen Photoinitiatoren und photolytischen Säurespendern stellt die vorliegende Erfindung eine beträchtliche Bereicherung der Technik und einen erheblichen Fortschritt dar.

Die nachstehend aufgeführten Beispiele sollen die vorliegende Erfindung näher erläutern. Prozentzahlen und Mengenverhältnisse sind, wenn nichts anderes angegeben ist, auf Gewichtseinheiten bezogen. Die Mengen sind gewöhnlich in Gewichtsteilen (Gt) angegeben.

### Beispiel 1

### Herstellung von 4-(4,6-Bis-trichlormethyl-s-triazin-2-yl)-benzoesäuremethylester (Methode a1)

16 Gt 4-Cyano-benzoesäuremethylester werden mit 86,6 Gt Trichloracetonitril und 3,2 Gt Aluminiumbromid unter Feuchtigkeitsausschluß gerührt. Die klare Lösung wird auf 24 bis 28 °C temperiert und dann unter Rühren Chlorwasserstoffgas eingeleitet, bis keine HCl-Aufnahme mehr erfolgt (ca. 2 bis 5 Stunden). Das Reaktionsprodukt verfestigt sich in dieser Zeit zusehends. Das Rühren wird eingestellt und die sirupöse Mischung bei Raumtemperatur 24 Stunden nachreagieren gelassen. Das gelbe, feste Reaktionsprodukt wird in 500 Gt Dichlormethan aufgenommen und zweimal mit je 250 Gt destilliertem Wasser gewaschen. Die organische Phase wird über Natriumsulfat getrocknet. Nach Abdampfen des Lösungsmittels wird der weiße Rückstand aus 250 Gt Ethanol umkristallisiert. Ausbeute: 41 Gt = 91 % d. Th. weißer Kristalle von 4-(4,6-Bis-trichlormethyl-s-triazin-2-yl)-benzoesäuremethylester. Smp.: 157 bis 158 °C.

| C₁₃H₇N₃Cl₆O₂ (449,9) | | | | |
|---|---|---|---|---|
| ber. | C 34,70 % | H 1,51 % | N 9,34 % | Cl 47,28 % |
| gef. | C 34,6 % | H 1,4 % | N 9,1 % | Cl 47,7 % |

Der als Ausgangsverbindung eingesetzte 4-Cyano-benzoesäuremethylester läßt sich wie folgt herstellen:
Zu 14 Gt Hydroxylammoniumchlorid werden 16 Gt Pyridin gegeben, und die Mischung wird gerührt. Dazu werden unter Rühren 32,8 Gt 4-Methoxycarbonyl-benzaldehyd gegeben, wobei sich die Mischung erwärmt. Nach 10 Minuten wird mit 200 Gt m-Xylol versetzt und das Gemisch an einem Wasserabscheider zum Rückfluß erhitzt. Nach etwa 10 Stunden hat sich die theoretische Menge Wasser abgeschieden. Das Gemisch wird auf Raumtemperatur abkühlen gelassen, mit 200 Gt Diethylether verdünnt und zweimal mit je 150 Gt destilliertem Wasser gewaschen. Die organische Phase wird mit Magnesiumsulfat getrocknet, und die Lösungsmittel werden am Rotationsverdampfer entfernt, wobei letzte Xylolreste unter vermindertem Druck abgezogen werden. Das erhaltene, hauptsächlich aus 4-Cyano-benzoesäuremethylester bestehende Rohprodukt wird aus 150 Gt Ethanol umkristallisiert.

### Beispiel 2

### Herstellung von 3-(4,6-Bis-trichlormethyl-s-triazin-2-yl)-benzoesäuremethylester (Methode a1)

70 Gt 3-Cyanobenzoesäuremethylester werden mit 376 Gt Trichloracetonitril und 13,9 Gt Aluminiumbromid wie in Beispiel 1, Stufe 2, beschrieben vermischt und mit Chlorwasserstoffgas versetzt, reagieren gelassen und aufgearbeitet. Nach Umkristallisieren aus Ethanol werden 160 Gt = 82 % weißer Kristalle von 3-(4,6-Bis-trichlormethyl-s-triazin-2-yl)-benzoesäuremethylester erhalten. Smp.: 115 bis 117 °C.

| C₁₃H₇N₃Cl₆O₂ (449,9) | | | | |
|---|---|---|---|---|
| ber. | C 34,70 % | H 1,51 % | N 9,34 % | Cl 47,28 % |
| gef. | C 34,4 % | H 1,5 % | N 9,4 % | Cl 47,1 % |

Der als Ausgangsverbindung eingesetzte 3-Cyanobenzoesäuremethylester kann wie folgt hergestellt werden:
240 Gt Thionylchlorid und 240 Gt Toluol werden vermischt und mit 140 Gt 3-Cyano-benzoesäure versetzt. Die Suspension wird unter Feuchtigkeitsausschluß gerührt und zum Rückfluß erhitzt. Nach etwa 5 Stunden ist die SO₂-Entwicklung beendet und die Lösung klar. Überschüssiges Thionylchlorid und Toluol werden abdestilliert und der Rückstand vorsichtig in 600 Gt Methanol gegossen. Das Gemisch wird 24 Stunden stehen gelassen und dann zur Vervollständigung der Fällung auf 0 °C gekühlt. Der Niederschlag aus 3-Cyano-benzoesäuremethylester wird abgesaugt und über Phosphorpentoxid getrocknet.

### Beispiel 3

### Herstellung von 4-(4,6-Bis-trichlormethyl-s-triazin-2-yl)-zimtsäuremethylester (Methode a1)

46 Gt 4-Cyano-zimtsäuremethylester werden unter Feuchtigkeitsausschluß und bei 24 bis 28 °C in einer Mischung aus 208,2 Gt Trichloracetonitril und 7,68 Gt Aluminiumbromid suspendiert. Die Mischung wird gerührt und bei konstanter Temperatur Chlorwasserstoff eingeleitet. Vorübergehend bildet sich eine fast klare Lösung, bald darauf beginnt das Gemisch jedoch sirupös zu werden. Die Chlorwasserstoffgaseinleitung wird nach 4 Stunden beendet, das Rühren wird eingestellt und das Gemisch 12 Stunden bei 28 °C gehalten. Die erhaltene feste Masse wird in 500 Gt Dichlormethan gelöst und zweimal mit 250 Gt Wasser gewaschen. Die organische Phase wird mit Calciumchlorid getrocknet, und das Lösungsmittel wird abgedampft. Der Rückstand wird aus einem 1:1-Gemisch aus Methanol und 2-Methoxy-ethanol umkristallisiert.
Ausbeute: 81,1 Gt = 72 %, weiße Nadeln mit einem Schmelzpunkt von 162 bis 163 °C.

| C₁₅H₉N₃O₂Cl₆ (475,9) | | | | |
|---|---|---|---|---|
| ber: | C 37,85 | H 1,91 | N 8,83 | Cl 44,69 |
| gef: | C 37,3 | H 1,7 | N 9,0 | Cl 45,2 |

Die als Ausgangsstoff eingesetzte 4-Cyanozimtsäure kann wie folgt hergestellt werden:
50 Gt 4-Cyanozimtsäure, 27,7 Gt absolutes Methanol, 200 Gt 1,2-Dichlorethan und 1,6 Gt Toluolsulfonsäure werden ca. 15 Stunden zum Rückfluß erhitzt. Die Kontrolle durch Dünnschichtchromatographie zu diesem Zeitpunkt zeigt, daß die Umsetzung vollständig abgelaufen ist. Die klare Lösung wird mit Eis gekühlt, worauf ein Teil des Produkts auskristallisiert und durch Filtration isoliert wird. Die Mutterlauge wird mit 5 %iger Natriumhydrogencarbonatlösung und zweimal mit Wasser gewaschen. Nach dem Trocknen der organischen Phase mit Magnesiumsulfat wird das Lösungsmittel abgezogen und das erhaltene Produkt über Phosphorpentoxid getrocknet. Die beiden Chargen von 4-Cyano-zimtsäuremethylester werden vereinigt, da beide gleiche Reinheit aufweisen.

### Beispiel 4

### Herstellung von 3-(4,6-Bis-trichlormethyl-s-triazin-2-yl)-zimtsäuremethylester (Methode a1)

42 Gt 3-Cyano-zimtsäuremethylester werden unter Feuchtigkeitsausschluß und bei ca. 25 °C zu einer Mischung aus 194,4 Gt Trichloracetonitril und 7,2 Gt Aluminiumbromid gegeben. Anschließend wird 3 Stunden lang unter Rühren Chlorwasserstoff eingeleitet, wobei die Temperatur konstant gehalten wird. Das Rühren wird beendet und die Mischung über Nacht nachreagieren gelassen. Auch hierbei wird eine konstante Temperatur von 25 °C eingehalten. Das verfestigte Gemisch wird in 800 Gt Dichlormethan gelöst, mit Wasser gewaschen und die organische Phase über Natriumsulfat getrocknet. Nach Abdampfen des Lösungsmittels erhält man einen weißen Rückstand, der aus Methanol oder Benzin umkristallisiert wird.
Ausbeute: 92 Gt = 86,3 % d. Th. weißer Nadeln mit einem Schmelzpunkt von 118,5 bis 119,5 °C.

| C₁₅H₉N₃O₂Cl₆ (475,9) | | | | |
|---|---|---|---|---|
| ber: | C 37,85 | H 1,91 | N 8,83 | Cl 44,69 |
| gef: | C 37,9 | H 1,9 | N 9,0 | Cl 44,9 |

Der eingesetzte 3-Cyano-zimtsäuremethylester kann wie folgt hergestellt werden:
105 Gt 3-Cyanozimtsäure, 58 Gt absolutes Methanol, 350 Gt 1,2-Dichlorethan und 2,2 Gt Toluolsulfonsäure werden 35 Stunden unter Rückfluß gekocht. Da auch nach dieser Zeit noch keine vollständige Umsetzung beobachtet wird, wird das Gemisch erkalten gelassen, zweimal mit 5 %-iger Natronlauge und anschließend mit Wasser gewaschen. Die organische Phase wird über Natriumsulfat getrocknet und der vom Lösungsmittel befreite Rückstand von 3-Cyano-zimtsäuremethylester über Phosphorpentoxid getrocknet.

### Beispiele 5 bis 15

Entsprechend den Beispielen 1 bis 4, werden folgende Verbindungen hergestellt (Methoden a1, a2 und a3; Methoden a2 und a3 entsprechen Methode a1 mit dem Unterschied, daß bei der Aufarbeitung nach Methode a2 die organische Phase mehrmals mit reichlich Wasser gewaschen wird; nach Methode a3 eine Trennung der Reaktionsmischung durch Hochvakuumdestillation erfolgt).

### Beispiel 5

4-(4,6-Bis-trichlormethyl-s-triazin-2-yl)-benzoesäureethylester, Smp. 101 bis 102 °C (Methode a1).

### Beispiel 6

3-(4,6-Bis-trichlormethyl-s-triazin-2-yl)-benzoesäureethylester, Smp 114,5 bis 115,5 °C (Methode a1).

### Beispiel 7

4-(4,6-Bis-trichlormethyl-s-triazin-2-yl)-zimtsäureethylester, Smp 134,5 bis 135,5 °C (Methode a1).

### Beispiel 8

3-(4,6-Bis-trichlormethyl-s-triazin-2-yl)-zimtsäureethylester, Smp 99 bis 100 °C (Methode a1).

### Beispiel 9

4-(4,6-Bis-trichlormethyl-s-triazin-2-yl)-benzoesäure-(2-chlor-ethylester), Smp. 108 bis 109 °C (Methode a1).

### Beispiel 10

4-(4,6-Bis-trichlormethyl-s-triazin-2-yl)-benzoesäurephenylester, Smp 148 bis 150 °C (Methode a1).

### Beispiel 11

4-(4,6-Bis-trichlormethyl-s-triazin-2-yl)-benzoesäure-(2-phenyl-ethylester), Smp 113 bis 115 °C (Methode a1).

### Beispiel 12

4-(4,6-Bis-trichlormethyl-s-triazin-2-yl)-benzoesäure, Smp. 275 °C (Methode a2).

### Beispiel 13

3-(4,6-Bis-trichlormethyl-s-triazin-2-yl)-zimtsäure, Smp. 210 bis 211 °C (Methode a2).

### Beispiel 14

4-(4,6-Bis-trichlormethyl-s-triazin-2-yl)-benzoesäurechlorid, Smp. 100 bis 101 °C (Methode a3).

### Beispiel 15

4-(4,6-Bis-trichlormethyl-s-triazin-2-yl)-zimtsäurechlorid, Smp. 156 bis 157 °C (Methode a3).

### Beispiel 16

### Herstellung von 4-(4,6-Bis-trichlormethyl-s-triazin-2-yl)-benzoesäure (Methode b)

125 Gt 4-(4,6-Bis-trichlormethyl-s-triazin-2-yl)-benzoesäuremethylester, 250 Gt Trichloressigsäure und 2 Gt konz. Schwefelsäure werden unter Rühren auf 175 °C erwärmt. Bei dieser Temperatur wird gebildeter Trichloressigsäuremethylester abdestilliert. Nachdem etwa 40 Gt Trichloressigsäuremethylester abdestilliert sind, wird ein leichtes Vakuum von 270 mbar angelegt, um die Destillation zu vervollständigen. Der sich verschiedentlich verfestigende Rückstand wird auf 80 °C abkühlen gelassen und dann in 1500 Gt Eiswasser gegeben. Das Gemisch wird 30 Minuten digeriert und anschließend das Produkt abgesaugt. Ausbeute: 87 Gt = 72 % d. Th., weiße Kristalle (aus Toluol) vom Smp 275 °C.

| C₁₂H₅N₃O₂Cl₆ (435,9) | | | | |
|---|---|---|---|---|
| ber: | C 33,06 | H 1,16 | N 9,64 | Cl 48,80 |
| gef: | C 33,3 | H 1,0 | N 9,6 | Cl 48,3 |

### Beispiel 17

### Herstellung von 3-(4,6-Bis-trichlormethyl-s-triazin-2-yl)-benzoesäure (Methode b)

Analog zu Beispiel 16 werden aus 120 Gt 3-(4,6-Bis-trichlormethyl-s-triazin-2-yl)-benzoesäuremethylester 106 Gt = 91 % d. Th. 3-(4,6-Bis-trichlormethyl-s-triazin-2-yl)-benzoesäure mit dem Schmelzpunkt 211,5 °C (Benzin/Toluol 10 : 1) erhalten.

| C₁₂H₅N₃O₂Cl₆ (435,9) | | | | |
|---|---|---|---|---|
| ber: | C 33,06 | H 1,16 | N 9,64 | Cl 48,80 |
| gef: | C 33,1 | H 1,0 | N 9,6 | Cl 49,2 |

### Beispiel 18

### Herstellung von 4-(4,6-Bis-trichlormethyl-s-triazin-2-yl)-zimtsäure (Methode b)

In einen mit trockenem Stickstoff gespülten Kolben werden 450 Gt trockenes 1,2-Dichlorethan, 49,2 Gt Hexamethyldisilan und 85,3 Gt Jod vorgelegt. Dazu werden portionsweise 80 Gt 4-(4,6-Bis-trichlormethyl-s-triazin-2-yl)-zimtsäuremethylester gefügt, wobei die gelegentlich exotherm ablaufende Reaktion durch Kühlung unter Kontrolle gehalten wird. Das Gemisch wird nach vollständiger Zugabe und unter Verwendung eines bei -20 °C arbeitenden Kühlersystems zum Rückfluß erhitzt und solange erwärmt, bis die Dünnschichtchromatographie eine praktisch vollständige Umsetzung anzeigt (4 bis 12 Stunden). Nach dem Erkalten wird das Reaktionsgemisch mit 400 Gt Wasser versetzt und am Rotationsverdampfer eingeengt, bis das Dichlorethan abgedampft ist. Die zurückbleibende wäßrige Mischung wird mit Methanol versetzt, wobei die Säure quantitativ ausfällt. Diese wird abgesaugt und aus Eisessig (umkristallisiertes Produkt enthält dann 1 Molekül Eisessig) oder einer Wasser/Eisessig-Mischung umkristallisiert. Ausbeute: 60,5 Gt = 78 % d. Th., Smp. 233 bis 234 °C

| C₁₄H₇N₃O₂Cl₆ (461,9) | | | | |
|---|---|---|---|---|
| ber: | C 36,40 | H 1,53 | N 9,10 | Cl 46,05 |
| gef: | C 36,2 | H 1,35 | N 8,9 | Cl 46,5 |

### Beispiel 19

### Herstellung von 3-(4,6-Bis-trichlormethyl-s-triazin-2-yl)-zimtsäure (Methode b)

Analog zu Beispiel 18 werden aus 80 Gt 3-(4,6-Bis-trichlormethyl-s-triazin-2-yl)-zimtsäuremethylester 67 Gt = 86 % d. Th. 3-(4,6-Bis-trichlormethyl-s-triazin-2-yl)-zimtsäure mit einem Schmelzpunkt von 210 bis 211 °C (aus Eisessig/Wasser) erhalten.

| C₁₄H₇N₃O₂Cl₆ (461,9) | | | | |
|---|---|---|---|---|
| ber: | C 36,40 | H 1,53 | N 9,10 | Cl 46,05 |
| gef: | C 36,1 | H 1,4 | N 8,9 | Cl 45,7 |

### Beispiel 20

### Herstellung von 4-(4,6-Bis-trichlormethyl-s-triazin-2-yl)-benzoesäurepropylester (Methode e)

30 Gt 4-(4,6-Bis-trichlormethyl-s-triazin-2-yl)-benzoesäure, 14 Gt n-Propanol, 220 Gt Chloroform und 1,8 Gt Toluolsulfonsäure werden an einem Wasserabscheider zum Rückfluß erhitzt. Nach etwa 12 Stunden scheidet sich kein Wasser mehr ab. Die Mischung wird erkalten gelassen, mit 400 Gt Ether verdünnt und zweimal mit 5 %iger Natriumhydrogencarbonatlösung und zweimal mit Wasser gewaschen. Nach Trocknung mittels Magnesiumsulfat werden die Lösungsmittel abdestilliert, und der Rückstand wird aus Ethanol umkristallisiert. Ausbeute: 25 Gt 4-(4,6-Bis-trichlormethyl-s-triazin-2-yl)-benzoesäurepropylester, Smp. 106 bis 108 °C.

| C₁₅H₁₁N₃O₂Cl₆ (477,9) | | | | |
|---|---|---|---|---|
| ber: | C 37,69 | H 2,32 | N 8,79 | Cl 44,50 |
| gef: | C 37,2 | H 2,1 | N 8,8 | Cl 44,6 |

### Beispiele 21 bis 25

Entsprechend Beispiel 20 werden folgende Esterderivate hergestellt (Methode e):

### Beispiel 21

4-(4,6-Bis-trichlormethyl-s-triazin-2-yl)-benzoesäure-(2-methoxy-ethyl)-ester, Smp. 69 bis 71 °C.

### Beispiel 22

4-(4,6-Bis-trichlormethyl-s-triazin-2-yl)-benzoesäureprop-(2)-in-yl-ester, Smp. 108 bis 110 °C.

### Beispiel 23

3-(4,6-Bis-trichlormethyl-s-triazin-2-yl)-benzoesäureethylester, Smp. 113 bis 115 °C.

### Beispiel 24

4-(4,6-Bis-trichlormethyl-s-triazin-2-yl)-zimtsäurepropylester, Smp. 127 bis 130 °C.

### Beispiel 25

3-(4,6-Bis-trichlormethyl-s-triazin-2-yl)-zimtsäureethylester, Smp. 100 °C.

### Beispiel 26

### Herstellung von 4-(4,6-Bis-trichlormethyl-s-triazin-2-yl)-benzoesäurechlorid (Methode c)

Zu 350 Gt Thionylchlorid werden 87 Gt 4-(4,6-Bis-trichlormethyl-s-triazin-2-yl)-benzoesäure gegeben. Beim Erwärmen zum Rückfluß unter Feuchtigkeitsausschluß und unter starkem Rühren ist eine deutliche SO₂-Entwicklung zu beobachten. Nach 6 Stunden liegt eine klare Lösung vor. Überschüssiges Thionylchlorid wird, zuletzt unter Anlegen von Vakuum, abdestilliert. Der Rückstand, der in praktisch quantitativer Ausbeute reines 4-(4,6-Bis-trichlormethyl-s-triazin-2-yl)-benzoylchlorid enthält, wird aus Hexan umkristallisiert, Smp. 100 bis 101 °C.

| C₁₂H₄N₃OCl₇ (454,3) | | | | |
|---|---|---|---|---|
| ber: | C 31,72 | H 0,89 | N 9,25 | Cl 54,62 |
| gef: | C 31,9 | H 0,7 | N 9,3 | Cl 54,7 |

### Beispiele 27 bis 30

Entsprechend Beispiel 26 werden folgende Säurechloride und -bromide hergestellt (Methode c):

### Beispiel 27

3-(4,6-Bis-trichlormethyl-s-triazin-2-yl)-benzoylchlorid, Smp. 102 bis 103 °C.

### Beispiel 28

4-(4,6-Bis-trichlormethyl-s-triazin-2-yl)-cinnamoylchlorid, Smp. 156 bis 158 °C.

### Beispiel 29

3-(4,6-Bis-trichlormethyl-s-triazin-2-yl)-cinnamoylchlorid, Smp. 177 bis 178,5 °C.

### Beispiel 30

4-(4,6-Bis-trichlormethyl-s-triazin-2-yl)-benzoylbromid. Keine exakte Schmelzpunktbestimmung möglich, da das Produkt extrem reaktiv ist und an der Luft rasch hydrolysiert. Durch Umsetzung mit absolutem Methanol wird der Methylester erhalten, Smp. 157 bis 158 °C.

### Beispiel 31

### Herstellung von 4-(4,6-Bis-trichlormethyl-s-triazin-2-yl)-benzoesäure (Methode d)

1 Gt 4-(4,6-Bis-trichlormethyl-s-triazin-2-yl)-benzoylchlorid wird in eine Mischung aus 10 Gt Tetrahydrofuran und 5 Gt Wasser gegeben. Zur Vervollständigung der Reaktion wird nach 6 Minuten 1 Gt Triethylamin zugegeben und weitere 10 Minuten gerührt. Das Lösungsmittel wird abdestilliert und der Rückstand aus Toluol/Benzin umkristallisiert. Das Produkt ist identisch mit den in den Beispielen 12 und 16 beschriebenen Verbindungen.

### Beispiel 32

### Herstellung von 4-(4,6-Bis-trichlormethyl-s-triazin-2-yl)-benzoesäurebutylester (Methode f)

Zu 100 Gt trockenem Toluol werden 9 Gt 4-(4,6-Bis-trichlormethyl-s-triazin-2-yl)-benzoylchlorid und 1 Gt n-Butanol gegeben. Die Mischung wird bei 15 °C gerührt und tropfenweise mit 1,9 Gt Pyridin versetzt. Es wird 3 Stunden nachgerührt. Die erhaltene Mischung wird mit 2 %iger Salzsäure, 2 %iger Natronlauge und dann mit Wasser gewaschen. Nach Trocknung der organischen Phase über Magnesiumsulfat wird das Lösungsmittel abdestilliert und der Rückstand aus Ethanol umkristallisiert. Smp. 109 bis 110 °C.

| C₁₆H₁₃N₃O₂Cl₆ (492,0) | | | | |
|---|---|---|---|---|
| ber: | C 39,06 | H 2,66 | N 8,54 | Cl 43,23 |
| gef: | C 38,6 | H 2,5 | N 8,6 | Cl 43,3 |

### Beispiele 33 bis 37

Analog Beispiel 32 werden folgende Esterderivate hergestellt (Methode f):

### Beispiel 33

3-(4,6-Bis-trichlormethyl-s-triazin-2-yl)-benzoesäuremethylester. Smp. 115 °C. Präparat identisch mit dem in Beispiel 2 beschriebenen Produkt.

### Beispiel 34

4-(4,6-Bis-trichlormethyl-s-triazin-2-yl)-benzoesäure-(2-phenoxy-ethyl)-ester, Smp. 116 bis 118 °C.

### Beispiel 35

4-(4,6-Bis-trichlormethyl-s-triazin-2-yl)-benzoesäure-(4-nitro-phenyl)-ester, Smp 192 bis 194 °C.

### Beispiel 36

4-(4,6-Bis-trichlormethyl-s-triazin-2-yl)-zimtsäurephenylester, Smp. 137 bis 141 °C.

### Beispiel 37

4-(4,6-Bis-trichlormethyl-s-triazin-2-yl)-zimtsäureethylester, Smp 98 bis 100 °C.

### Anwendungsbeispiel 38

Eine Platte aus elektrolytisch aufgerauhtem und anodisiertem Aluminium wird mit einer Beschichtungslösung aus
6,7 Gt Trimethylolethan-triacrylat,
6,5 Gt eines Copolymeren aus Methylmethacrylat und Methacrylsäure, Säurezahl 115,
0,15 Gt der Verbindung des Beispiels Nr. 37,
64,0 Gt 2-Methoxy-ethanol,
22,7 Gt Butylacetat und
0,3 Gt 2,4-Dinitro-6-chlor-2′-acetylamino-5′-methoxy-4′-(N-β-hydroxyethyl-N-β-cyanoethyl-amino)-azobenzol
schleuderbeschichtet, so daß sich nach dem Trocknen ein Schichtgewicht von 3,5 g/m² ergibt. Anschließend wird die Platte mit einem Überzug aus Polyvinylalkohol versehen und unter einer Strich- und Rastervorlage mit einer 5kW-Metallhalogenidlampe 40 Sekunden lang belichtet. Die belichtete Schicht wird mit einer 1,5 %igen Lösung von Natriummetasilikat entwickelt.

Man erhält eine negative Abbildung der Vorlage. Auch feinste Raster- und Strichelemente sind klar wiedergegeben. Beim Einspannen der Platte in eine Bogenoffsetmaschine erhält man 185.000 qualitativ einwandfreie Drucke.

### Anwendungsbeispiel 39

20,6 Gt 5-(3,4-Dimethoxy-phenyl)-tetrazol-(2H) in 200 Gt Pyridin werden mit 48 Gt 4-(4,6-Bis-trichlormethyl-s-triazin-2-yl)-zimtsäurechlorid versetzt und langsam zum Rückfluß erwärmt. Die Lösung färbt sich unter Stickstoffentwicklung dunkel. Nach 2 Stunden wird in Eiswasser gegossen. Das Produktgemisch wird mit wäßriger Salzsäure schwach sauer gestellt und mit einer 1:1-Mischung aus Diethylether und Tetrahydrofuran ausgeschüttelt und mit Magnesiumsulfat getrocknet. Nach Abdampfen der Lösungsmittel wird das Gemisch in Toluol aufgenommen, wobei ein gelber Rückstand in diesem Lösungsmittel nicht löslich ist. Dieser wird durch Filtration isoliert und erweist sich als 2-[4-(4,6-Bis-trichlormethyl-s-triazin-2-yl)-phenylethenyl]-5-(3,4-dimethoxy-phenyl)-1,3,4-oxadiazol von Smp. 241,5 bis 242 °C.

Eine Beschichtungslösung entsprechend Anwendungsbeispiel 38 wird derart abgeändert, daß anstelle der Verbindung des Beispiels Nr. 37 0,25 Gt der vorstehend beschriebenen Verbindung verwendet werden. Nach Belichtung und Entwicklung erhält man eine sauber druckende Offsetplatte, die auch nach 200.000 Drucken einwandfreie Qualitäten liefert.

### Anwendungsbeispiel 40

22,7 Gt 3-(4,6-Bis-trichlormethyl-s-triazin-2-yl)-benzoesäurechlorid und 15,5 Gt N-Ethyl-2-methyl-benzthiazolium-p-toluolsulfonat werden in 150 Gt Toluol suspendiert. Zu dieser Mischung werden bei 15 °C 13,1 Gt Triethylamin zugetropft. Das Gemisch wird 4 Stunden nachgerührt, abgesaugt, mit Methanol und anschließend mit Wasser gewaschen und dann aus Acetonitril umkristallisiert. Man erhält gelbe Kristalle von 2-[3-(4,6-Bis-trichlormethyl-s-triazin-2-yl)-benzoylmethylen]-N-ethyl-benzthiazolin.

Eine Beschichtungslösung entsprechend Beispiel 38 wird derart verändert, daß die Verbindung des Beispiels 37 durch 0,25 Gt der vorstehend beschriebenen Verbindung ersetzt wird.

Nach der im Anwendungsbeispiel 38 beschriebenen Verarbeitung erhält man ein negatives Abbild der Vorlage, das auch bei hohen Druckauflagen keine Abrieberscheinungen aufweist.

### Anwendungsbeispiel 41

Eine Beschichtungslösung entsprechend Anwendungsbeispiel 38 wird derart abgeändert, daß anstelle der Verbindung Nr. 37 folgende Mischung in den angegebenen Mengen verwendet wird:
0,25 Gt der Verbindung Nr. 1
0,22 Gt Dibenzalaceton (Sensibilisator)
Die zubereitete lichtempfindliche Lösung wird auf ein elektrochemisch vorbehandeltes Blech aufgebracht und zu einem Trockenschichtgewicht von 2,2 g/m² getrocknet. Anschließend wird die Schicht mit einer Schutzschicht aus Polyvinylalkohol überzogen. Eine derart erhaltene Platte wird 30 Sekunden mit einer Metallhalogenidlampe durch eine Vorlage mit feinen Strichelementen und einem Halbtonstufenkeil belichtet und anschließend mit dem in Beispiel 38 angegebenen Entwickler entwickelt. Zum Vergleich wird eine Platte unter einem Kantenfilter, das die Lichtanteile unterhalb 400 nm (Kantenfilter 1) ausblendet, durch die oben genannte Vorlage belichtet, wobei die Belichtungszeit um den Faktor 2 erhöht wird. Anschließend erfolgt die Entwicklung.

Es werden folgende durchgehärtete Stufen beobachtet:

| Gesamtbelichtung | Kantenfilter 1 |
|---|---|
| 4 - 5 | 4 - 5 |

Der erfindungsgemäße Initiator läßt sich demnach durch geeignete Sensibilisatoren für Licht geringerer Energie anregen.

### Anwendungsbeispiel 42

Es wird wie im Anwendungsbeispiel 41 vorgegangen, jedoch wird das dort angegebene Initiatorgemisch durch
0,25 Gt der Verbindung Nr. 1
0,3 Gt Eosin
0,6 Gt eines Umsetzungsproduktes aus 1 Teil Triethanolamin und 3 Teilen Butylisocyanat
ersetzt. Zusätzlich zu den oben aufgeführten Belichtungen wird durch ein Kantenfilter belichtet, das die Lichtanteile unterhalb 450 nm (Kantenfilter 2) ausblendet. Die Belichtungszeit wird gegenüber der Standardbelichtung um den Faktor 5 erhöht. Nach der Entwicklung werden folgende Ergebnisse erhalten:

| Gesamtbelichtung | Kantenfilter 1 | Kantenfilter 2 |
|---|---|---|
| 5 | 4 - 5 | 4 - 5 |

Daraus ergibt sich ebenfalls, daß der erfindungsgemäße Initiator durch geeignete Sensibilisierung gegenüber sichtbarem Licht aktiviert werden kann.

## Patentansprüche

1. Verbindungen der allgemeinen Formel I 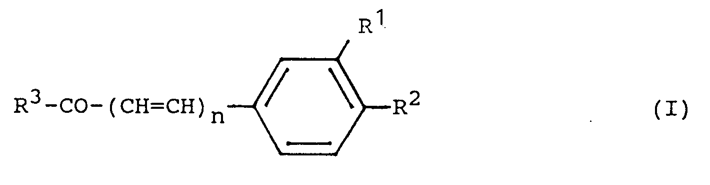 worin
R¹ und R² voneinander verschieden sind und entweder ein Wasserstoffatom oder einen Rest der Formel 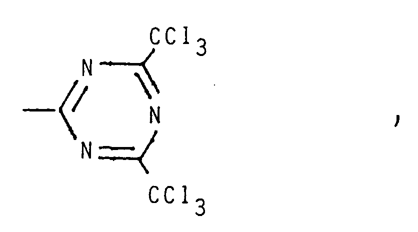
R³ einen ggf. durch Halogenatome oder Arylgruppen substituierten Alkoxyrest mit 1 bis 16 Kohlenstoffatomen, einen Alkenyloxy- oder Alkinyloxyrest mit 2 bis 6 Kohlenstoffatomen, einen Alkoxyalkoxyrest mit 3 bis 10 Kohlenstoffatomen, einen ggf. durch Halogenatome, Alkyl-, Alkoxy- oder Nitrogruppen substituierten Aryloxyrest mit 6 bis 14 Kohlenstoffatomen oder einen Aryloxyalkoxyrest mit 8 bis 12 Kohlenstoffatomen, eine Hydroxygruppe oder ein Halogenatom und
n die Zahl 0 oder 1
bedeuten.

2. Verbindungen gemäß Anspruch 1, dadurch gekennzeichnet, daß R³ einen Alkoxyrest mit 1 bis 6 Kohlenstoffatomen, einen Alkenyloxyrest oder Alkinyloxyrest mit 2 bis 4 Kohlenstoffatomen, eine Hydroxygruppe oder ein Chloratom bedeutet.

3. Verfahren zur Herstellung von Verbindungen der allgemeinen Formel I 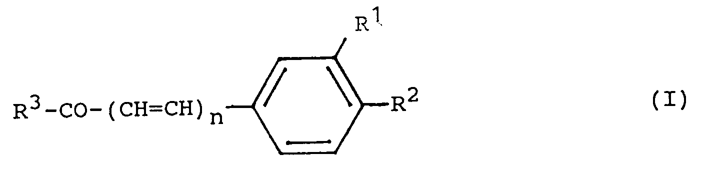 worin R¹, R², R³ und n die im Anspruch 1 angegebene Bedeutung haben, dadurch gekennzeichnet, daß man eine Verbindung der Formel II 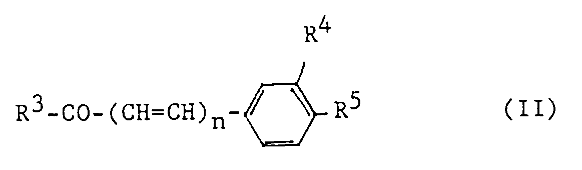 worin R⁴ und R⁵ voneinander verschieden sind und entweder ein Wassertoffatom oder eine CN-Gruppe bedeuten, mit Trichloracetonitril unter Einwirkung von HCl-Gas in Gegenwart einer Lewis-Säure umsetzt.

4. Verfahren nach Anspruch 3, dadurch gekennzeichnet, daß man eine Verbindung der Formel II einsetzt, in der R³ ein Chloratom ist oder zusammen mit der Carbonylgruppe eine Carbonsäureestergruppe bildet.

5. Verfahren nach Anspruch 3, dadurch gekennzeichnet, daß man als Lewis-Säure ein Aluminiumtrihalogenid einsetzt.

6. Verwendung von Verbindungen gemäß Anspruch 1 als Photoinitiatoren in lichtempfindlichen Gemischen.

## Claims

1. A compound of the general formula I 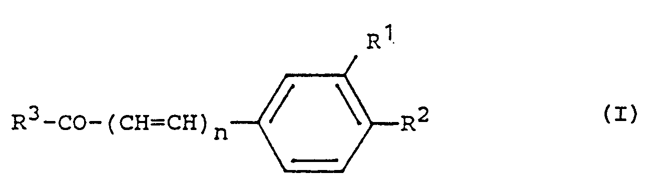 in which
R¹ and R² are different from one another and denote either a hydrogen atom or a radical of the formula 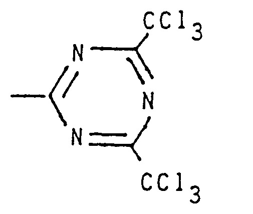
R³ denotes an alkyl radical having 1 to 16 carbon atoms which is unsubstituted or substituted by halogen atoms or aryl groups, an alkenyloxy or alkynyloxy radical having 2 to 6 carbon atoms, an alkoxyalkoxy radical having 3 to 10 carbon atoms, an aryloxy radical having 6 to 14 carbon atoms which is unsubstituted or substituted by halogen atoms, alkyl, alkoxy or nitro groups, or an aryloxyalkoxy radical having 8 to 12 carbon atoms, a hydroxyl group or a halogen atom and
n denotes the number 0 or 1.

2. A compound as claimed in claim 1, wherein
R³ denotes an alkoxy radical having 1 to 6 carbon atoms, an alkenyloxy radical or alkynyloxy radical having 2 to 4 carbon atoms, a hydroxyl group or a chlorine atom.

3. A process for the preparation of compounds of the general formula I 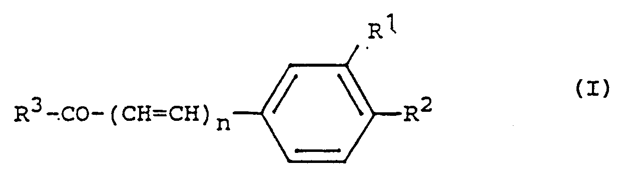 in which R¹, R², R³ and n have the meaning given in claim 1, which comprises reacting a compound of the formula II 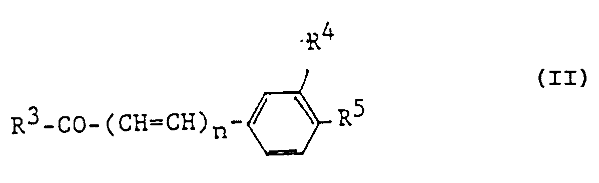 in which R⁴ and R⁵ are different from one another and denote either a hydrogen atom or a CN group, with trichloroacetonitrile under the influence of HCl gas in the presence of a Lewis acid.

4. The process as claimed in claim 3, wherein a compound of the formula II is used in which R³ is a chlorine atom or together with the carbonyl group forms a carboxylic ester group.

5. The process as claimed in claim 3, wherein the Lewis acid used is an aluminum trihalide.

6. Use of the compound as claimed in claim 1 as photoinitiators in light-sensitive mixtures.

## Revendications

1. Composés de formule générale I 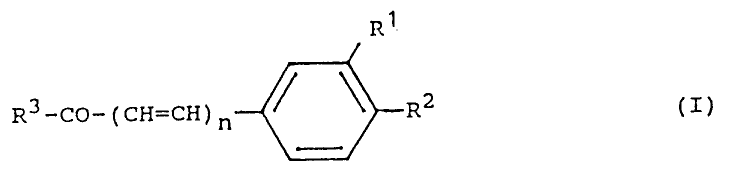 dans laquelle
R¹ et R² sont différents l'un de l'autre et représentent soit un atome d'hydrogène, soit un radical de formule 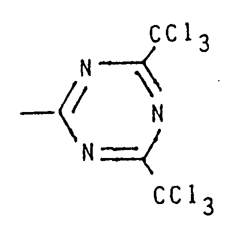
R³ représente un radical alcoxy ayant de 1 à 16 atomes de carbone, éventuellement substitué par des atomes d'halogène ou des groupes aryle, un radical alcényloxy ou alcynyloxy ayant de 2 à 6 atomes de carbone, un radical alcoxyalcoxy ayant de 3 à 10 atomes de carbone, un radical aryloxy ayant de 6 à 14 atomes de carbone, éventuellement substitué par des atomes d'halogène, des groupes alkyle, alcoxy ou nitro, ou un radical aryloxyalcoxy ayant de 8 à 12 atomes de carbone, le groupe hydroxy ou un atome d'halogène, et
n représente le nombre 0 ou 1.

2. Composés selon la revendication 1, caractérisés en ce que R³ représente un radical alcoxy ayant de 1 à 6 atomes de carbone, un radical alcényloxy ou un radical alcynyloxy ayant de 2 à 4 atomes de carbone, le groupe hydroxy ou un atome de chlore.

3. Procédé pour la préparation des composés de formule générale I 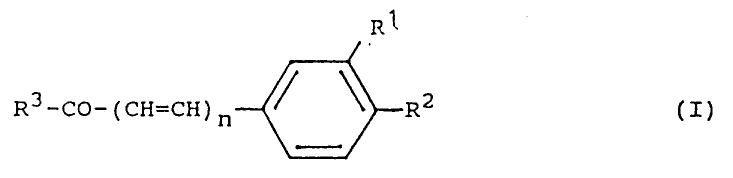 dans laquelle R¹, R², R³ et n ont les significations données dans la revendication 1, caractérisé en ce que l'on fait réagir un composé de formule II 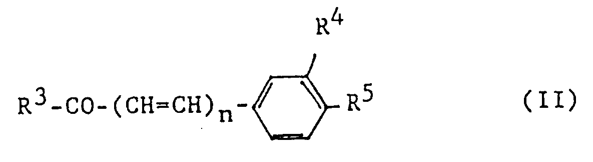 dans laquelle R⁴ et R⁵ sont différents l'un de l'autre et représentent soit un atome d'hydrogène, soit le groupe CN, avec du trichloracétonitrile sous l'effet de gaz chlorhydrique, en présence d'un acide de Lewis.

4. Procédé selon la revendication 3, caractérisé en ce que l'on utilise un composé de formule II dans lequel R³ est un atome de chlore ou forme, conjointement avec le groupe carbonyle, un groupe ester d'acide carboxylique.

5. Procédé selon la revendication 3, caractérisé en ce que, comme acide de Lewis, on utilise un trihalogénure d'aluminium.

6. Utilisation des composés selon la revendication 1 en tant que photoinitiateurs dans des compositions photosensibles.
